## Europäisches Patentamt
## European Patent Office
## Office européen des brevets

(19)

(11) Publication number: **0 030 063**
**B1**

(12)

# EUROPEAN PATENT SPECIFICATION

(45) Date of publication of patent specification: **24.08.83**

(21) Application number: **80201128.8**

(22) Date of filing: **27.11.80**

(51) Int. Cl.³: **C 12 N 7/00,**
**A 61 K 39/215,**
**A 61 K 39/295**

(54) Infectious-bronchitis vaccines for poultry, combined infectious-bronchitis vaccines, process for preparing such vaccines, process for preventing infectious bronchitis and infectious-bronchitis virus strains.

(30) Priority: **30.11.79 NL 7908687**
**09.09.80 NL 8005083**

(43) Date of publication of application:
**10.06.81 Bulletin 81/23**

(45) Publication of the grant of the patent:
**24.08.83 Bulletin 83/34**

(84) Designated Contracting States:
**AT BE CH DE FR GB IT LI LU NL SE**

(56) References cited:
**GB - A - 1 137 306**
**GB - A - 1 174 125**
**US - A - 2 998 349**
**US - A - 3 876 763**

**BIOLOGICAL ABSTRACTS, vol. 63, June 1,
1977, abstract 63802
Philadelphia, PA, US
G. MEULEMANS et al. "Isolation of a new
serotype of the avian infections bronchitis virus
in Belgium"**

(73) Proprietor: **Gist - Brocades N.V.**
**Wateringseweg 1 P.O. Box 1**
**NL-2600 MA Delft (NL)**

(72) Inventor: **Apontowell, Peter**
**Burg. Martenslaan 39**
**NL-3956 EK Leersum (NL)**
Inventor: **Krasselt, Manfred Max**
**Park Arenberg 59**
**NL-3731 EP De Bilt (NL)**

(74) Representative: **Van der Straaten, Jan Anthony
et al,**
**c/o GIST-BROCADES N.V. Patents and
Trademarks Department Wateringseweg 1 P.O.
Box 1**
**NL-2600 MA Delft (NL)**

(56) References cited:
**BIOLOGICAL ABSTRACTS, vol. 59, no. 1,
January 1975, abstract 3108
Philadelphia, Pa. US
K. OTSUKI et al. "Preparation and immunilogical
response to a new mixed vaccine composed of
inactivated Newcastle disease virus, inactivated
infectious bronchitis virus, and inactivated
Hemophilus gallinarum"**

(56) References cited:

BIOLOGICAL ABSTRACTS, vol. 57, no. 5, May 1974 abstract 26709
Philadelphia, Pa. US
R. B. JOHNSON et al. "A new serotype of infectious bronchitis virus responsible for respiratory disease in Arkansas broiler flocks"

BIOLOGICAL ABSTRACTS, vol. 57, no. 5, May 1974, abstracts 26711
Philadelphia, Pa. US
L. VAN DER HEIDE et al. "Infectious bronchitis outbreaks in vaccinated poultry in Connecticut"

BIOLOGICAL ABSTRACTS, vol. 62, September 15, 1976 abstract 32419
Philadelphia, Pa. US
R. B. JOHNSON et al. "New strains of infectious bronchitis virus iodated in Maine"

BIOLOGICAL ABSTRACTS, vol. 62, July 15 1976, abstract 8254. R. W. WINTERFIELD et al. "Immunity to infectious bronchitis virus from spray vaccination with derivatives of a Holland strain"

Infectious-bronchitis vaccines for poultry, combined infectious-bronchitis vaccines, process for preparing such vaccines, process for preventing infectious bronchitis and infectious-bronchitis virus strains.

The invention is relating to infectious bronchitis (IB) vaccines for poultry and a process for the preparation of such vaccines and more particularly to improved infectious bronchitis vaccines, derived from at least one infectious bronchitis virus strain of a novel serotype as to those of the virusses, which are known up to now for vaccination purposes.

The application of live infectious bronchitis vaccines for poultry is already known for many years.

Infectious bronchitis is an important affection of the respiratory system, the kidneys and oviduct of poultry. The cause of this syndrome is a corona virus. The poultry is severely affected by epizootics of this disease.

The infectious bronchitis still causes a high mortality, especially in young poultry. Besides the mortality and more or less strong respiratory symptoms, lesions to the oviducts and as a result thereof egg production drops occur caused by an IB infection.

Moreover infections with IB virus may stimulate latent virus- or bacterial infections and may give rise in this way to severe economical losses, especially in the broiler field.

For the combatment of infectious bronchitis as well vaccines, derived from inactivated virus as those ones derived from live virus, are applied. However, it was found that a loss of immunogenic properties occurred after inactivation of these viruses with e.g. formaline and ultra violet light (M. S. Hofstad, Diseases of Poultry, Biester and Schwarte, Iowa University, Press. Ames (1965), 615).

As also normal, sound chickens can be killed or diseased by primary vaccination with a live, non or less attenuated virus vaccine, whereby an especial danger is existing for animals of less than 2 or 3 weeks old or for chickens shortly before the start of or during laying, people skilled in this art have a clear preference for the application of dead vaccines or of live vaccines, whereby was tried to increase the harmlessness of such vaccines by means of attenuation of the original IB field virus isolates.

For example the H-strain, which is presently applied on world wide scale on account of his broad immunisation spectre against among others Massachusetts and Connecticut type IB-virus and which was isolated and attenuated by Bijlenga, Hoekstra and Rispens, is disclosed in Tijdschr.Diergeneesk. *81*:43, "Infectious bronchitis in chicks in the Netherlands" (1956), Tijdschr.Diergeneesk. *85*:320 (1960), Tijdschr.Diergeneesk. *85*:279 (1960) and Tijdschr.Diergeneesk 85:398 (1960).

For such modified vaccines, viruses, having undergone 25 or more embryo passages to reduce their pathogenicity and their disseminating ability, are applied up to now, such as e.g. the Massachusetts type and more particularly the IBV W 48, M 41, 82828 besides the H52 and H120 strains thereof, these two having been passaged approximately 52 and 120 times respectively on chicken embryonated eggs, a Connecticut isolate, e.g. A 5968 or the Beaudette IBV type (IBV—42). The immunizing capacity of these viruses is very specific against Massachusetts or Connecticut types of IB viruses.

Although the use of vaccines of these modified strains has presently appeared to be safe and effective, these vaccines have appeared to be still unable to prevent outbreaks of infectious bronchitis in a sufficient way under certain conditions, as appears from Avian Diseases vol. 20, no. 1, p. 42 and 177 and Avian Diseases vol. 19, no. 2, p. 323 and 583.

This shortcoming of the present IB vaccines is attributed to occurring antigenic variations of the virus in an important degree, as also appears e.g. from Archiv für die Gesamte Virusforschung *34*, p. 32 (1971) and Cunningham C. H. Develop. Biol. Standard, *33*, 311 (1976).

Efforts were made therefore to reach an adequate vaccination of poultry by means of preparation and application of combined vaccines, derived from several IBV strains of different serotypes.

However, hereby a clearly encountered difficulty appeared to form the decrease of immunogenic properties of the respective starting viruses, caused by mutual interaction, as appears from Am. J. Vet. Res. *36*, 4, 524 and 525 (1965) and Avian Diseases *12*, 577 (1968).

Therefore there is still existing a large need for IB vaccines with adequate immunizing properties.

It will be appreciated that the pursued improvement of these vaccines are still severely hampered, caused by changing immunogenic and other properties of the presently available IB viruses after a large number of passages in embryonated chicken eggs, the appearance of new serotypes and the lack of sufficiently effectively applicable serological and immunological test procedures respectively.

In this connection reference may be made to Avian Diseases, *19, 2*, 323 and 324 (1975).

As a result of extensive research and experimentation, a number of novel IB viruses could surprisingly be obtained, which can be regarded as deviating from the up to now most frequently applied IB viruses of the H type (e.g. IB H120 and IB H52) in cross neutralization tests (virus neutralization tests) according to e.g. the method as described in American Association of Avian Pathologists, "Isolation and Identification of Avian Pathogens", page 184 (1975), in the understanding that antisera diluted in a ratio of 1:5 are applied, and in challenge experiments with subsequent virusreisolation tests. In other words at an inoculation with a virus of the H-type (e.g. IB H120 and IB H52) the concerning animals are not protected against virusreplication in the mucosa of the respiratory system after a challenge with the beforementioned deviating novel IB viruses.

3

O 030 063

Humoral antibodies against the IB H strain equally appeared not to be able to neutralize significant amounts of IB virus of the mentioned deviating types.

Of special importance for the practice is that these novel IB viruses cause respiratory symptoms with animals showing high antibody titers against the IB H strain, and with still laying animals, egg production drops.

Examples of such novel viruses are those ones, identified by the internal notations. Utrecht.101 (U.101), Utrecht.102 (U.102) , Drente.201 (D.201), Limburg.501 (L.501), Limburg.502 (L.502), Brabant.801 (B.801), Limburg 536 (L.536), Overijssel.728 (O.728) and Utrecht.121 (U.121) as deposited at the Czechoslovak National Collection of Type Cultures of the Institute of Hygiene and Epidemiology in Prague under the respective nos. CNCTC A07/80, CNCTC A08/80, CNCTC A09/80, CNCTC A010/80, CNCTC A011/80 (on March 6, 1980), CNCTC A016/80, CNCTC A014/80, CNCTC A015/80 and CNCTC A013/80 (on September 9, 1980) and at the Collection Nationale de Cultures de Microorganismes d'Institut Pasteur, Paris, under the resp. nos. I—111, I—112, I—113, I—109, I—110 (on November 14, 1979) and I—132, I—134, I—135 and I—133 (on October 3, 1980).

These viruses were isolated by means of the trachea swab method with flocks of laying chickens, which had been vaccinated two and three times respectively with IB H120 and IB H52 vaccine and which showed high humoral antibody titers as to the Massachusetts type of the IB virus at the beginning of the occurrence of respiratory symptoms and/or egg production drops and with broilers, which showed in the second half of the fattening period, respiratory symptoms after previous vaccinations with IB vaccine of the H120 type, whereby both kinds of animals found themselves at the moment of isolation in districts, which have been indicated in the hereinbefore used internal notations.

There was now found that by attenuation to SPF-chickenembryos, the isolated virusstrains have lost their pathogenicity for SPF chickens in a major degree, in spite of the fact that their immunising ability has remained still present.

For example the virusstrain with internal notation IBV Utrecht.101 showed these beforementioned characteristics after 85 SPF type I chickenembryo passages and the virusstrain IBV Limburg.536 showed these characteristics after 56 SPF type I chickenembryo passages.

There was surprisingly found during a comparing test, using conventional H120 or H52 vaccines, that the protection against IB viruses of the Massachusetts type — measured as the amount of virus neutralizing antibodies — had not been diminished in a significant degree, if a H-type vaccine combined with e.g. the novel IBV isolate Utrecht 101 was administered instead of the H-type vaccine. During this experimentation one started from intranasal application of the concerning vaccines.

For instance the following neutralization indices were determined 4 weeks after administration of the respective vaccines and vaccine combinations.

TABLE 1

Immunising ability of different vaccines measured on the humoral immune response (neutralization-index)

| Vaccine | Testvirus /N.I.H. (B222) | IBV U. 101 | IBV L. 536 |
|---|---|---|---|
| 1. H120 | 6.5 | 1.7 | 0.8 |
| 2. IBV U. 101 (82nd egg passage) | 2.1 | 6.8 | N.D. |
| 3. H120 + IBV U. 101 | 6.2 | 6.5 | N.D. |
| 4. IBV L. 536 (53rd egg passage) | 0.9 | N.D. | 5.8 |
| 5. H120 + IBV L. 536 | 6.7 | N.D. | 5.7 |

In addition to the immuno response after administation of different vaccines and vaccine combinations respectively, the resistance against virusreplication and persistence was also determined in and on the mucosa of the trachea, determined by means of the virus reisolation technique, as described in "Specifications for the production and control of avian live virus vaccines" of the Ministry of Agriculture, Fisheries of Food of the United Kingdom Central Veterinary Laboratory of Biological Products and Standards Department, New Haw, Weybridge, Surrey KT 153 NB, 2nd Edition (1977), p. 12.

4

Cross neutralization tests in SPF chicken embryos and cross infection tests on SPF chickens were carried out according to the hereunder following tables:

TABLE 2

Cross neutralization test in SPF chickenembryos

| Antiserum against virustype | Virusisolate | | | | |
|---|---|---|---|---|---|
| | H | U. 101 | 0.728 | L. 536 | |
| H | $\geqslant 7,2$ | neg. | neg. | neg. | |
| U. 101 | neg. | 5,3 | neg. | neg. | N.I. $(10^X)$ |
| O. 728 | neg. | neg. | 5,8 | neg. | |
| L. 536 | neg. | neg. | neg. | $\geqslant 6,2$ | |

In this connection with the expression "neg." is meant, that no significant positive neutralization indexes (N.I.), i.e. N.I.$\leqslant 10^{2.0}$ are obtained.

TABLE 3

Cross challenge tests carried out on SPF chickens;
results of virusresolationtests.

| Vaccine virus | Challenge virus | | | |
|---|---|---|---|---|
| | "VOET" type Massachusetts | U. 101 | 0.728 | L. 536 |
| H 120th egg-passage | neg. | + | + | + |
| U. 101 50th egg-passage | + | neg. | + | + |
| O. 0.827 100th egg-passage | + | + | neg. | + |
| L. 536 80th egg-passage | + | + | + | neg. |

With the term "neg." is meant in this connection that none of the SPF eggs, injected with the tracheaswab material, showed symptoms which may be attributed to an infection with IB virus.

From the results of tables 2 and 3 (neutralization and crosschallenge tests) it appears that the four applied serotypes of the IB virus, namely those of the H-, U.101-, 0.728-and L.536-type, not only differ antigenitically from each other, but additionally — having in mind the results of the challenge experiments with additional virusreisolation — show attractive immunogenic properties as to the homologeous virus.

Field experiments showed that in sera of broilers, reproduction chickens and laying hens, antibodies were frequently occurring against the virustypes Utrecht.101, Limburg.536 and Overijssel.728.

It will be appreciated therefore that the novel IB virustypes are not only differing antigenitically in a significant degree from the usually applied H-virus, but moreover are showing significantly different properties as to each other.

The novel isolated virusstrains could be characterized by the following tests:

Treatment of the infectious amnion allantoic fluid obtained by cultivation of original virus containing samples from infected homogenized organ and tracheaswabmaterial in the allantoic hole of 10 days prebrooded SPF eggs with chloroform according to Mayr, A. et al, Virologische Arbeitsmethoden G. Fischer Verlag, Jena, 1977, p. 285 resulted, in comparison with the non-treated material, in a reduction of the repeatedly measured viruscontent from $10^{7.5}$ to $10^{1.5}$ $EID_{50}$. This experience may point to the presence of a virus agent, which is containing in his envelope a lipide, which is necessary for the infectivity.

The infectious amnion-allantoic fluid caused no agglutination with erythrocytes derived from SPF chickens.

Addition of 5-fluordesoxyuridine (FUDR) to the culture medium of chicken kidney cell cultures, serving for replication of the agent, did not influence the intracellular synthesis of the virus agent in a significant degree.

The $EID_{50}$ content of the cellmaterial and culture medium appeared to reside on comparable levels 2, 4 and 7 days after the inoculation of the virus agent, i.e. the nucleic acid to be replicated belonged to the group of the ribonucleic acid.

Examination with electron microscope showed, that the virus agent, present in the amnion allantoic fluid which was harvested within 30 hours after the artificial infection, possessed a diameter of about 100 nm. About 15 nm long projections were present on the surface of this virus. The virus has the size and shape of a corona virus, to which also the avian bronchitisviruses are regarded to belong.

It will be appreciated that the properties of the novel serotypes of IB-viruses as described hereinbefore make the novel virusstrains especially suitable for the preparation of as well inactivated as live poultry vaccines on behalf of a more efficient protection against infectious bronchitis, especially in areas or countries, wherein the described deviating serotypes according to the present invention occur besides the IB viruses of the socalled H-type.

More particularly virusstrains of the serotypes of the hereinbefore mentioned novel virusstrains may successfully be applied for the preparation of mixed live and inactivated vaccines, derived as well from the novel IB strains as from the H-strains with one or more of the novel IB-strains.

The novel IBV vaccines of the present invention may be obtained by propagation of one or more novel virus strains by methods known in the art in principle and optionally followed by inactivation by methods known in the art in principle.     For instance the virus may be propagated in embryonated SPF chicken eggs or in suitable cell cultures such as chicken kidney cell cultures. However with such a process there has to be checked whether the antigenic properties do not significantly change.

Hereafter the cultivated virus material is collected and purified. At last one or more stabilizers and possibly antibiotics, such as sodium penicillin G, streptomycin or natamycin, may be added and the mixture is lyophilized.

More particularly, the concerning seed virus is inoculated under sterile conditions into the allantoic cavity in 10—11 days prebrooded chicken eggs of type I SPF.

After incubation for 28 to 34 hours at 37°C, the amnion-allantoic fluid of the then still living and of the specifically died (i.e. after 24 hours after the seed virus inoculation) embryos is harvested, purified and lyophilized after optional addition of stabilizers and/or antibiotics.

According to this process, single vaccines could be prepared, which contained the virus after lyophilisation in an amount of $\geqslant 10^{4.0}$ $EID_{50}$ per dose, while e.g. so prepared combined vaccines of a novel virusstrain and a known H-strain or of more novel virusstrains showed a virus content of $\geqslant 2 \times 10^{4.0}$ $EID_{50}$ per dose and preferably a content of each of the virus components of $\geqslant 10^{4.0}$ $EID_{50}$ per dose.

It will be appreciated that the present invention is also relating to novel, as well inactivated as live, IBV vaccines, which have been at least derived from one of the novel IB virusstrains and to the application of such vaccines.

Preferably live vaccines, derived from one or more of the novel viruses or from the H-type virus with one or more of the novel IB-viruses, are applied.

More preferably live vaccines, derived from H120 or H52 virusstrain and from one or more IB viruses, selected from the group consisting of U.101, L.536 and O.728, are applied. The vaccines may also be applied with young animals and more preferably with broilers.

The vaccines may be administered by the socalled eyedrop- or nosedrop, the drinking-water or spraymethod for live vaccines.

Vaccination by means of novel live vaccines according to the present invention has preferably to be carried out with poultry of an age of 1 day to 18 weeks. Novel inactivated vaccines are subcutaneously or intramuscularly administered to animals.

It will be appreciated that also combined live or inactivated vaccines, derived from at least one of the novel IB virustypes and one or more completely other virustypes, such as e.g. the Newcastle disease virus, adeno- or reo viruses, form a feature of the present invention too.

For the preparation of invactivated IBV vaccines according to the present invention there may be started from e.g. an amnion-allantoic fluid, to which a suitable carrier is added, after inactivation by methods known in the art, e.g. by means of beta-propiolactone or formaline.

Preferably the virus liquid of a suitable titer is processed to a water in oil emulsion vaccine,

# 0 030 063

derived from a mineral or plant oil and one or more emulsifiers, such as non-ionic surface-active compounds derived from alkylene oxide and/or hexahydric alcohols and/or higher natural fatty acids ($C_{10}$—$C_{20}$) such as esters or ester-ethers.

Examples of the lastmentioned emulsifiers are mannide monooleate (Span®80, Arlacel 80®, Arlacel A®, and polyoxyethylene (20) sorbitan monooleate (e.g. Tween 80®).

The volume ratio between the aqueous phase (virus fluid) and the oily phase may vary from 3:7 to 1:1 and lies preferably at a ratio of about 7:13.

The invention is illustrated by the following examples, however without any restriction of the object of the invention to these specific embodiments.

## Example 1
### Preparation of IB virus vaccine of the strain U.101

A. Cultivation of virus.

Type I SPF chicken eggs, which were preincubated 10 to 11 days, are inoculated with $10^{3.0}$ to $10^{4.0}$ $EID_{50}$ IBV U.101 seed virus (0.2 ml pro egg) in the allantoic cavity.

The eggs are inspected for the first time after 20 to 24 hours from the virusinoculation and all aspecific died embryos are removed.

After an incubationperiod of in total 28 hours at +37°C, the amnion-allantoic fluid (AAF) is harvested.

B. Treatment of virus suspension.

After purification of the AAF by means of centrifugation at 2000 r.p.m. in a cooling centrifuge and/or filtration, 5 × $10^5$ units of sodium penicillin G and 800 mg of streptomycin per liter are added to this AAF.

The virusmaterial is subsequently stabilized by addition of at least 3% by weight of albumin and/or mannitol.

The stabilized bulk virusmaterial is frozen to at least −35°C and stored at such temperature until the further processing phase.

Samples of this material are meanwhile tested on their virus content by means of the $EID_{50}$ (Egg Infection Dose 50%) assay method.

After the test results have become available, the virus material is thawn up again and filled out into lyophilization flasks.

The viruscontent (volume) is adjusted hereby in such a way, that at the end of the subsequent lyophilization there are still at least $10^{4.5}$ $EID_{50}$ of the concerning virus per dose present in the vaccine.

The flasks are sealed under vacuum at the end of the lyophilization.

With the preparation of the multivalent (mixed) vaccine care has to be taken that the minimal virus contents for all viruscomponents are reached.

## Example 2
### Preparation of IB virus vaccine of the strain L.536.

A. Cultivation of virus.

Type I SPF chicken eggs, which have been preincubated during 10 to 11 days, are inoculated with $10^{3.0}$ to $10^{4.0}$ $EID_{50}$ IBV L.536 seed virus (0.2 ml pro egg) into the allantoic cavity.

The eggs are inspected for the first time and all aspecifically died embryos are removed after 20 to 24 hours from the virus inoculation. After an incubation period of in total 32 hours at +37°C, the AAF is harvested.

B. Treatment of the virus suspension.

After purification of the AAF by means of centrifugation at 2000 r.p.m. in a cooling centrifuge and/or filtration, 6 × $10^5$ units of sodium penicillin G and 900 mg of streptomycin per liter are added to this AAF.

The virus material is subsequently stabilized by addition of about 5% by weight of albumin and/or mannitol. As albumin e.g. bovine albumin may be applied. The stabilized bulk virus material is subsequently frozen to at least −35°C and kept at such temperature until the further processing phase.

Meanwhile samples of this material are tested on their virus content by means of the $EID_{50}$ (Egg Infectious Dose 50%) assay method. The virus material is thawn up again after the testresults have become available and filled out into lyophilisationflasks.

The virus content (volume) is adjusted hereby in such a way that at the end of the lyophilisation process at least $10^{4.5}$ $EID_{50}$ of the concerning virus per dose are present in the vaccine. The flasks are sealed under vacuum at the end of the lyophilisation.

During the preparation of the multivalent (mixed) vaccine care has to be taken that the minimum virus content for all virus components is reached.

## Example 3
### Preparation of IB-virus vaccine of the strain 0.728.

A. Cultivation of virus.

Type I SPF chicken eggs, which have been preincubated during 10 to 11 days, are inoculated with

7

$10^{3.0}$ to $10^{4.0}$ $EID_{50}$ IBV 0.728 seed virus (0,2 ml per egg).

The eggs are inspected for the first time and all aspecifically died embryos are removed after 20 to 24 hours from the virus inoculation. After an incubation period of in total 32 hours at +37°C, the AAF is harvested.

B. Treatment of the virus suspension.

After purification of the AAF by means of centrifugation at 2000 r.p.m. in a cooling centrifuge and/or filtration, 8 x $10^5$ units of sodium penicillin G and 1100 mg of streptomycine per liter are added to the AAF.

The virus material is subsequently stabilized by the addition of about 7% by weight of albumine and/or mannitol.

The stabilized bulk virus material is subsequently frozen to at least −35°C and kept at this temperature until the further processing phase. Meanwhile samples of this material are tested on their virus content by means of the $EID_{50}$ assay method.

The virus material is thawn up again and filled out into lyophilisationflasks after the testresults have become available.

The virus content (volume) is adjusted hereby in such a way that at the end of the lyophilisation process at least $10^{4.5}$ $EID_{50}$ of the concerning virus per dosis are present in the vaccine. The flasks are sealed under vacuum at the end of the lyophilisation.

During the preparation of the multivalent (mixed) vaccine care has to be taken that the minimum virus content for all virus components is reached.

Example 4

Preparation of a combined IB-virus vaccine of the strains U.101, L.536 and 0.728.

A. Cultivation of virus.

Type I SPF chicken eggs, which have been preincubated during 10 to 11 days, are inoculated with $10^{3.0}$ to $10^{4.0}$ $EID_{50}$ IBV U.101, L.536 and 0.728 seed virus (0,2 ml in total per egg) into the allantoic cavity.

The eggs are inspected for the first time and all aspecifically died embryos are removed after 20 to 24 hours from the virus inoculation. After an incubation period of in total 32 hours at 37°C, the AAF is harvested.

B. Treatment of the virus suspension.

After purification of the AAF by means of centrifugation at 2000 r.p.m. in a cooling centrifuge and/or filtration, 8 x $10^5$ units of sodium penicillin G and 1000 mg of streptomycine per liter are added to this AAF.

The virus material is subsequently stabilized by addition of at least 3% by weight of albumine and/or mannitol.

The stabilized bulk virus material is subsequently frozen to at least −35°C and kept at this temperature until the further processing phase.

Meanwhile samples of this material are tested on their virus content by means of the $EID_{50}$ assay method.

The virus material is thawn up again and filled out into lyophilisationflasks after the testresults have become available.

The virus content (volume) is adjusted hereby in such a way that at the end of the lyophilisation process at least $10^{4.5}$ $EID_{50}$ of each concerning virus per dosis is present in the vaccine. The flasks are sealed under vacuo at the end of the lyophilisation.

During the preparation of the multivalent (mixed) vaccine care has to be taken that the minimum virus content for all virus components is reached.

Example 5

Preparation of inactivated combined IB-virus vaccine of the strains H.52, U.101, L.536 and 0.728.

In a similar way as described in example 4.A., the virus was cultivated in SPF eggs and the obtained virus suspension was treated in a similar way as in example 4.B., until the frozen phase is reached, however, without addition of antibiotics and stabilizers.

The frozen AAF is defrosted and inactivated in a water bath by 0.1% of β-propiolactone during 90 minutes at 37°C.

The virus suspension is kept overnight at +4°C. The inactivation is checked by inoculation of prebrooded embryonated SPF chicken eggs with the inactivated virus material and subsequent incubation.

The inactivated AAF is diluted if necessary, with PBS + 0,3% of formaline, dependent on the $EID_{50}$ of each virus type, determined in the non-inactivated AAF (at least $10^{7.0}$ for all virusstrains).

To the virus suspension of the four strains 3.5% of Tween 80® are added.

The inactivated virus suspension is mixed with an oil phase in the ratio of 6.5 parts of oil/3.5 parts of virus fluid and emulsified, so that the average particle size of the aqueous phase is about 0.5 $\mu$.

The emulsifying is carried out by means of an Ultra Turrax homogeniser or by means of passing the starting mixture through a colloid+mill.

8

## 0 030 063

The applied oily phase has the following composition:

| | |
|---|---|
| Marcol 5 2® (white paraffinic Esso oil) | 93,5% |
| Arlacel A®, Arlacel 80® or Span 80® (mannide monooleate) | 6,5% |

The components of the oily phase are separately heated to 110°C in an autoclave or the mixture is sterily filtrated.

**Claims for the Contracting States: BE CH LI DE FR GB IT LU NL SE**

1. Infectious bronchitis vaccines for poultry, characterized in that they are derived from at least one virus strain of a novel serotype of the infectious bronchitis viruses, indicated by the internal notation Utrecht.101, Utrecht.102, Drente.201, Limburg.501, Limburg.502, Brabant.801, Limburg.536, Overijssel.728 and Utrecht.121, which have been deposited with the Czechoslovak National Collection of Type Cultures of the Institute of Hygiene and Epidemiology in Prague under the nos. CNCTC A 07/80, CNCTC A 08/80, CNCTC A 09/80, CNCTC A 010/80, CNCTC A 011/80, CNCTC A 016/80, CNCTC A 014/80, CNCTC A 015/80 and CNCTC A 013/80; and with the Collection Nationale de Cultures de Microorganismes d'Institut Pasteur, Paris, under the respective nos. I—111, I—112, I—113, I—109, I—110, I—132, I—134, I—135 and I—133.

2. Infectious bronchitis vaccines, according to claim 1, characterized in that they are derived from at least one virus strain of a novel serotype of the infectious bronchitis virus, indicated by the internal notations Utrecht.101, Limburg.536 and Overijssel.728, which have been deposited with the Czechoslovak National Collection of Type Cultures of the Institute of Hygiene and Epidemiology in Prague under the nos. CNCTC A 07/80, CNCTC A 014/80 and CNCTC A 015/80 and with the Collection Nationale de Cultures de Microorganismes d'Institut Pasteur under no. I—111, I—134 and I—135.

3. Combined infectious bronchitis vaccines, according to the claims 1 and 2, characterized in that these vaccines in addition are derived from the IBV H120 or the IBV H52 of the Massachusetts type.

4. Infectious bronchitis vaccine, according to claim 3, characterized in that it is derived in addition from the IBV H52 of the Massachusetts type.

5. Live infectious bronchitis vaccines, according to claim 5, characterized in that they show after lyophilisation a virus content of $\geqslant 10^{4.0}$ EID$_{50}$ per dose.

6. Live infectious bronchitis vaccines, according to claim 5, characterized in that they show after lyophilisation a total virus content of $\geqslant 2 \times 10^{4.0}$ EID$_{50}$ per dose of each of the virus strains.

7. Inactivated infectious bronchitis vaccine, according to claims 1—4, characterized in that it contains an oily phase, containing as essential ingredients at least a mineral or plant oil and one or more suitable emulsifying agents in the form of a non-ionogenic surface active agent, derived from alkylene oxide and/or hexahydric alcohols and/or higher natural fatty acids of 10—20 carbon atoms, such as esters or ester-ethers.

8. Infectious bronchitis virus strains of a novel serotype indicated by means of the internal notations Utrecht.101, Utrecht.102, Drente.201, Limburg.501, Limburg.502, Brabant.801, Limburg.536, Overijssel.728 and Utrecht.121, which have been deposited with the Czechoslovak National Collection of Type Cultures of the Institute of Hygiene and Epidemiology in Prague under the nos. CNCTC A 07/80, CNCTC A 08/80, CNCTC A 09/80, CNCTC A 010/80, CNCTC A 011/80, CNCTC A 016/80, CNCTC A 014/80, CNCTC A 015/80 and CNCTC A 013/80; and with the Collection Nationale de Cultures de Microorganismes d'Institut Pasteur, Paris, under the respective nos. I—111, I—112, I—113, I—109, I—110, I—132, I—134, I—135 and I—133.

9. Infectious bronchitis virus strain, indicated by means of Utrecht.101, Limburg.536 and Overijssel.728 and deposited at the Czechoslovak National Collection of Type Cultures of the Institute of Hygiene and Epidemiology in Prague under no. CNCTC A 07/80, CNCTC A 014/80 and CNCTC A 015/80 and at the Collection Nationale de Cultures de Microorganismes d'Institut Pasteur, Paris, under no. I—111, I—134 and I—135.

10. Process for the preparation of infectious bronchitis vaccines for poultry according to claim 1, characterized in that at least one of the virus strains is propagated as seed virus in fertilized chicken eggs or in suitable cell cultures such as SPF chicken kidney cell cultures, whereafter the cultivated virus material is collected and purified, followed by lyophilisation of the mixture after the optional addition of stabilizers and/or antibiotics.

11. Process according to claim 10, characterized in that the seed virus is inoculated under sterile conditions in the allantoic hole of type I SPF chicken eggs, which have been preincubated for 10—11 days, whereafter the amnion-allantoic fluid of still living and specifically died embryos is harvested after incubation at +37°C during 28 to 34 hours, purified and after addition of stabilizers and optionally antibiotics, lyophilized.

12. Process according to claims 10 and 11, characterized in that single vaccines are prepared,

9

which are containing after lyophilisation $\geqslant 10^{4.0}$ EID$_{50}$ per dose of the concerning virus.

13. Process for the preparation of combined vaccines according to claims 3 and 4, characterized in that the virus fluid, obtained by cultivation of at least one virus strain of the novel serotype of the infectious bronchitis viruses indicated by the internal notation Utrecht.101, Limburg.501, Limburg.502, Utrecht.102, Drente.201, Brabant.801, Limburg.536, Overijssel.728 and Utrecht.121, and subsequent purification and mixing, after optional addition of stabilizers and/or antibiotics, with a virus fluid, obtained by cultivation of an IBV H120 or an IBV H52, purification and optional addition of stabilizers and optional antibiotics, followed by lyophilisation.

14. Process for the preparation of combined vaccines according to claim 13, characterized in that vaccines are prepared, which are containing after lyophilisation a virus content being $\geqslant 10^{4.0}$ EID$_{50}$ per dose and preferably $\geqslant 2 \times 10^{4.0}$ EID$_{50}$ per dose of each of the virus components.

15. Inactivated infectious bronchitis vaccine, according to claim 7, characterized in that it is derived from besides one or more IBV strains in addition from one or more totally different virus types such as Newcastle disease virus, adenolike virus and/or reo virus.

## Claims for the Contracting State: AT

1. Process for the preparation of infectious bronchitis vaccines for poultry, characterized in that at least one virus strain of a novel sterotype of infectious bronchitis viruses, indicated by the internal notation Utrecht.101, Utrecht.102, Drente.201, Limburg.501, Limburg.502, Brabant. 801, Limburg.536, Overijssel.728 and Utrecht.121, which have been deposited at the Czechoslovak National Collection of Type Cultures of the Institute of Hygiene and Epidemiology in Prague under the nos. CNCTC A07/80, CNCTC A08/80, CNCTC A09/80, CNCTC A010/80, CNCTC A011/80, CNCTC A016/80, CNCTC A014/80, CNCTC A015/80 and CNCTC A013/80 and at the Collection Nationale de Cultures de Microorganismes d'Institut Pasteur, Paris under nos. I.111, I.112, I.113, I.109, I.110, I.132, I.134, I.135 and I.133, is propagated as seed virus by methods known in the art in principle, whereafter the cultivated virus material is collected and purified, followed by lyophilisation of the mixture after the optional addition of stabilizers and/or antibiotics and/or inactivation by methods known in principle.

2. Process according to claim 1, characterized in that the seed virus is propagated in fertilized chicken eggs or in suitable cell cultures, e.g. SPF chicken kidney cell cultures.

3. Process according to claims 1 and 2, characterized in that the seed virus is inoculated under sterile conditions in the allantoic hole of type I SPF chicken eggs, which have been preincubated for 10—11 days, whereafter the amnion-allantoic fluid of still living and specifically died embryos is harvested after incubation at +37°C during 28 to 34 hours, purified and after addition of stabilizers and optionally antibiotics, lyophilized.

4. Process according to claims 1—3, characterized in that single vaccines are prepared, which are containing after lyophilisation $\geqslant 10^{4.0}$ EID$_{50}$ per dose of the concerning virus.

5. Process according to claims 1—4, characterized in that vaccines are prepared derived from the IBV strains Utrecht.101, Limburg.536 or Overijssel.728.

6. Process for the preparation of combined vaccines according to claims 1—5, characterized in that the virus fluid, obtained by propagation of at least one virus strain of the novel serotype of the infectious bronchitis viruses indicated by the internal notation Utrecht.101, Limburg.501, Limburg.502, Utrecht.102, Drente.201, Brabant.801, Limburg.536, Overijssel.728 and Utrecht.121, and subsequent purification is mixed, after optional addition of stabilizers and/or antibiotics, with a virus fluid, obtained by cultivation of an IBV H.120 or an IBV H.52, purification and optional addition of stabilizers and optional antibiotics, followed by inactivation and/or lyophilisation.

7. Process for the preparation of combined vaccines according to claim 6, characterized in that vaccines are prepared, which are containing after lyophilisation a virus content being $\geqslant 10^{4.0}$ EID$_{50}$ per dose for each of the virus components.

8. Process for the preparation of combined vaccines according to claims 6—7, characterized in that the vaccines are derived from at least an IBV strain selected from Utrecht.101, Limburg.536 and Overijssel.728.

9. Process for the preparation of inactivated, combined ready for use, vaccines, characterized in that a virusfluid derived from one or more of the IBV virusstrains of the novel serotypes is mixed after inactivation, with one or more inactivated virusfluids derived from a totally different virustype such as Newcastle disease virus, adenolike virus and/or reo virus.

10. Live infectious bronchitis vaccines, obtained according to the processes of claims 1—8.

11. Inactivated infectious bronchitis vaccines, obtained according to the processes of claims 1—9.

12. Process for the preparation of an IBV strain of a novel serotype indicated by the internal notation Utrecht.101, Utrecht.102, Drente.201, Limburg.501, Limburg.502, Brabant.801, Limburg.536, Overijssel 728 and Utrecht.121, which have been deposited at the Czechoslovak National Collection of Type Cultures of the Institute of Hygiene and Epidemiology in Prague under the nos. CNCTC A07/80, CNCTC A08/80, CNCTC A09/80, CNCTC A010/80, CNCTC A011/80, CNCTC A016/80, CNCTC A014/80, CNCTC A015/80 and CNCTC A013/80 and at the Collection Nationale de Cultures de Microorganismes d'Institut Pasteur, Paris, under nos. I.111, I.112, I.113, I.109, I.110, I.132, I.134, I.135 and I.133, by isolation by means of the trachea swab method with flocks of chickens which have been vaccinated with the usual IBV H.120 and/or H.52 vaccine and which show high humoral antibody titers as to the Massachusetts type virus at the beginning of the occurrence of respiratory symptoms and/or egg production drops or with broilers in the second half of the fattening period showing respiratory symptoms after vaccination with an IBV vaccination of H type, and attenuation to SPF chickenembryos of such virus.

**Patentansprüche für die Vertragsstaaten: BE CH LI DE FR GB IT LU NL SE**

1. Infektiöse-Bronchitis-Vakzinen für Geflügel, dadurch gekennzeichnet, daß sie von zumindest einem Virusstamm eines neuen Serotyps der infektiöse-Bronchitis-Viren abgeleitet sind, die mit der internen Bezeichnung Utrecht.101, Utrecht.102, Drente.201, Limburg.501, Limburg.502, Brabant.801, Limburg.536, Overijssel.728 und Utrecht.121 gekennzeichnet sind und die hinterlegt worden sind in der Tschechoslowakischen Nationalen Kultursammlung des Instituts für Hygiene und Epidemiologie in Prag unter den Nummern CNCTC A 07/80, CNCTC A 08/80, CNCTC A 09/80, CNCTC A 010/80, CNCTC A 011/80, CNCTC A 016/80, CNCTC A 014/80, CNCTC A 015/80 und CNCTC A 013/80 und in der Collection Nationale de Cultures de Microorganismes d'Institut Pasteur, Paris, unter den Nummern I—111, I—112, I—113, I—109, I—110, I—132, I—134, I—135 und I—133.

2. Infektiöse-Bronchitis-Vakzinen nach Anspruch 1, dadurch gekennzeichnet, daß sie von mindestens einem Virusstamm eines neuen Serotyps des infektiöse-Bronchitis-Virus abgeleitet sind, wobei diese Stämme durch die internen Bezeichnungen Utrecht.101, Limburg.536 und Overijssel.728 gekennzeichnet und in der Tschechoslowakischen Nationalen Kultursammlung des Instituts für Hygiene und Epidemiologie in Prag unter den Nummern CNCTC A 07/80, CNCTC A 014/80 und CNCTC A 015/80 und in der Collection Nationale de Cultures de Microorganismes d'Institut Pasteur unter den Nummern I—111, I—134 und I—135 hinterlegt worden sind.

3. Kombinierte infektiöse-Bronchitis-Vakzinen nach den Ansprüchen 1 und 2, dadurch gekennzeichnet, daß diese Vakzinen zusätzlich vom IBV H120 oder dem IBV H52 des Massachussets-Typs abgeleitet sind.

4. Infektiöse-Bronchitis-Vakzine nach Anspruch 3, dadurch gekenzeichnet, daß sie zusätzlich von dem IBV H52 des Massachusetts-Typs abgeleitet ist.

5. Infektiöse-Bronchitis-Lebend-Vakzinen nach Anspruch 5, dadurch gekennzeichnet, daß sie nach Lyophilisation einen Virusgehalt von $\geqslant 10^{4,0}$ $EID_{50}$ je Dosis aufweisen.

6. Infektiöse-Bronchitis-Lebend-Vakzinen nach Anspruch 5, dadurch gekennzeichnet, daß sie nach Lyophilisation einen Gesamtvirusgehalt von $\geqslant 2 \times 10^{4,0}$ $EID_{50}$ je Dosis von jedem der Virusstämme aufweisen.

7. Inaktivierte infektiöse-Bronchitis-Vakzine nach den Ansprüchen 1 bis 4, dadurch gekennzeichnet, daß sie eine ölige Phase enthält, die als wesentliche Bestandteile mindestens ein mineralisches oder pflanzliches Öl und ein oder mehrere geeignete Emulgiermittel in Form eines nichtionogenen, oberflächenaktiven Stoffes, abgeleitet von Alkylenoxid und/oder sechswertigen Alkoholen und/oder höheren natürlichen Fettsäuren mit 10 bis 20 Kohlenstoffatomen, wie Estern oder Ester-Äthern, enthält.

8. Infektiöse-Bronchitis-Virusstämme eines neuen Serotyps, gekennzeichnet durch die internen Bezeichnungen Utrecht.101, Utrecht.102, Drente.201, Limburg.501, Limburg.502, Brabant.801, Limburg.536, Overijssel.728 und Utrecht.121, und hinterlegt in der Tschechoslowakischen Nationalen Kultursammlung des Instituts für Hygiene und Epidemiologie in Prag unter den Nummern CNCTC A 07/80, CNCTC A 08/80, CNCTC A 09/80, CNCTC A 010/80, CNCTC A 011/80, CNCTC A 016/80, CNCTC A 014/80, CNCTC A 015/80 und CNCTC A 013/80 und in der Collection Nationale de Cultures de Microorganismes d'Institut Pasteur, Paris, unter den Nummern I—111, I—112, I—113, I—109, I—110, I—132, I—134, I—135 und I—133.

9. Infektiöse-Bronchitis-Virusstamm, bezeichnet als Utrecht.101, Limburg.536 und Overijssel.728 und hinterlegt in der Tschechoslowakischen Nationalen Kultursammlung des Instituts für Hygiene und Epidemiologie in Prag unter den Nummern CNCTC A 07/80, CNCTC A 014/80 und CNCTC A 015/80 und in der Collection Nationale de Cultures de Microorganismes d'Institut Pasteur, Paris, unter den Nummern I—111, I—134 und I—135.

10. Verfahren zur Herstellung von infektiöse-Bronchitis-Vakzinen für Geflügel nach Anspruch 1, dadurch gekennzeichnet, daß mindestens einer der Virusstämme als Saatvirus in befruchteten Hühnereiern oder in geeigneten Zellkulturen, wie SPF-Hühnernieren-Zellkulturen, vermehrt wird, worauf das kultivierte Virusmaterial abgetrennt und gereinigt wird, gefolgt von einer Gefriertrocknung der Mischung, gewünschtenfalls nach Zusatz von Stabilisatoren und/oder Antibiotika.

11. Verfahren nach Anspruch 10, dadurch gekennzeichnet, daß das Saatvirus unter sterilen Bedingungen in die Allantois-Höhle von SPF-Hühnereiern des Typs I, die 10 bis 11 Tage vorbebrütet worden sind, eingeimpft wird, worauf die Amnion-Allantois-Flüssigkeit von noch lebenden und spezifisch abgestorbenen Embryonen nach Inkubation bei $+37°C$ während 28 bis 34 h geerntet, gereinigt und nach Zusatz von Stabilisatoren und gewünschtenfalls Antibiotika gefriergetrocknet wird.

12. Verfahren nach den Ansprüchen 10 und 11, dadurch gekennzeichnet, daß Einzel-Vakzinen hergestellt werden, die nach Lyophilisation $\geqslant 10^{4,0}$ EID$_{50}$ je Dosis des betreffenden Virus enthalten.

13. Verfahren zur Herstellung von kombinierten Vakzinen nach den Ansprüchen 3 und 4, dadurch gekezeichnet, daß die Virusflüssigkeit, die durch Kultivieren von mindestens einem Virusstamm des neuen Serotyps der infketiöse-Bronchitis-Viren, die durch die interne Bezeichnung Utrecht.101, Limburg.501, Limburg.502, Utrecht.102, Drente.201, Brabant.801, Limburg.536, Overijssel.728 und Utrecht.121 gekennzeichnet sind, und nachfolgende Reiningung erhalten wurde, nach einem gewünschtenfalls erfolgenden Zusatz von Stabilisatoren und/oder Antibiotika, mit einer Virusflüssigkeit, die durch Kultivieren eines IBV H120 oder eines IBV H52, Reinigung und gewünschtenfalls Zusatz von Stabilisatoren und gewünschtenfalls Antibiotika, gefolgt von einer Lyophilisation, erhalten wurde, vermischt wird.

14. Verfahren zur Herstellung von kombinierten Vakzinen nach Anspruch 13, dadurch gekennzeichnet, daß Vakzinen hergestellt werden, die nach Lyophilisation einen Virusgehalt von $\geqslant 10^{4,0}$ EID$_{50}$ je Dosis und vorzugsweise $\geqslant 2 \times 10^{4,0}$ EID$_{50}$ je Dosis an jeder Viruskomponente aufweisen.

15. Inaktivierte infektiöse-Bronchitis-Vakzine nach Anspruch 7, dadurch gekennzeichnet, daß sie außer von einem oder mehreren IBV—Stämmen zusätzlich noch von einem oder mehreren gänzlich verscheidenen Virustypen, wie Newcastle Disease-Virus, einem Adeno-ähnlichen Virus und/oder Reo-Virus, abgeleitet ist.

**Patentansprüche für den Vertragsstaat: AT**

1. Verfahren zur Herstellung von infektiöse-Bronchitis-Vakzinen für Geflügel, dadurch gekennzeichnet, daß mindestens ein Virusstamm eines neuen Serotyps von infektiöse-Bronchitis-Viren, gekennzeichnet durch die interne Bezeichnung Utrecht.101, Utrecht.102, Drente.201, Limburg.501, Limburg.502, Brabant.801, Limburg.536, Overijssel.728 und Utrecht.121, die hinterlegt worden sind in der Tschechoslowakischen Nationalen Kultursammlung, Institut für Hygiene und Epidemiologie in Prag unter den Nummern CNCTC A07/80, CNCTC A08/80, CNCTC A09/80, CNCTC A010/80, CNCTC A011/80, CNCTC A016/80, CNCTC A014/80, CNCTC A015/80 und CNCTC A013/80 und in der Collection Nationale de Cultures de Microorganismes d'Institut Pasteur, Paris, unter den Nummern I.111, I.112, I.113, I.109, I.110, I.132, I.134, I.135 und I.133, als Saatvirus mit im Prinzip auf diesem Gebiet bekannten Methoden vermehrt wird, worauf das kultivierte Virusmaterial abgetrennt und gereinigt wird, gefolgt von einer Gefriertrock-nung der Mischung nach einem gewünschtenfalls erfolgten Zusatz von Stabilisatoren und/oder Antibiotika und/oder Inaktivierung durch im Prinzip bekannte Methoden.

2. Verfahren nach Anspruch 1, dadurch gekennzeichnet, daß das Saatvirus in befruchteten Hühnereiern oder in geeigneten Zellkulturen, z.B. SPF-Hühnernieren-Zellkulturen, vermehrt wird.

3. Verfahren nach Anspruch 1 und 2, dadurch gekennzeichnet, daß das Saatvirus unter sterilen Bedingungen in die Allantois-Höhle von SPF-Hühnereiern des Typs I, die 10 bis 11 Tage vorbebrütet worden sind, eingeimpft wird, worauf die Amnion-Allantois-Flüssigkeit von noch lebenden und spezifisch abgestorbenen Embryos nach Inkubation bei $+37\,^{\circ}$C während 28 bis 34 h geerntet, gereinigt und nach Zusatz von Stabilisatoren und gewünschtenfalls Antibiotika gefriergetrocknet wird.

4. Verfahren nach den Ansprüchen 1 bis 3, dadurch gekennzeichnet, daß Einzel-Vakzinen hergestellt werden, die nach Lyophilisation $\geqslant 10^{4,0}$ EID$_{50}$ je Dosis des betreffenden Virus enthalten.

5. Verfahren nach den Ansprüchen 1 bis 4, dadurch gekennzeichnet, daß Vakzinen abgeleitet von den IBV—Stämmen Utrecht.101, Limburg.536 oder Overijssel.728 hergestellt werden.

6. Verfahren zur Herstellung von kombinierten Vakzinen nach den Ansprüchen 1 bis 5, dadurch gekennzeichnet, daß die Virusflüssigkeit, die durch Vermehrung von mindestens einem Virusstamm des neuen Serotyps der infektiöse-Bronchitis-Viren, die durch die interne Bezeichnung Utrecht.101, Limburg.501, Limburg.502, Utrecht.102, Drente.201, Brabant.801, Limburg.536, Overijssel.728 und Utrecht.121 gekennzeichnet sind, und nachfolgende Reinigung erhalten wurde, gewünschtenfalls nach Zusatz von Stabilisatoren und/oder Antibiotika, mit einer Virusflüssigkeit vermischt wird, die durch Kultivieren eines IBV H120 oder eines IBV H52, Reinigung und gewünschtenfalls Zusatz von Stabilisatoren und gewünschtenfalls Antibiotika, gefolgt von einer Inaktivierung und/oder Lyophilisation, erhalten wurde.

7. Verfahren zur Herstellung von kombinierten Vakzinen nach Anspruch 6, dadurch gekennzeichnet, daß Vakzinen hergestellt werden, die nach Gefriertrocknung einen Virusgehalt von $\geqslant 10^{4,0}$ EID$_{50}$ je Dosis an jeder der Viruskomponenten aufweisen.

8. Verfahren zur Herstellung von kombinierten Vakzinen nach den Ansprüchen 6 und 7, dadurch gekennzeichnet, daß die Vakzinen von mindestens einem IBV-Stamm, ausgewählt aus Utrecht.101, Limburg.536 und Overijssel.728, abgeleitet sind.

9. Verfahren zur Herstellung von inaktivierten, kombinierten, gebrauchsfertigen Vakzinen, dadurch gekennzeichnet, daß eine Virusflüssigkeit, die von einem oder mehreren der IBV—Virusstämme der neuen Serotypen abgeleitet ist, nach Inaktivierung mit einer oder mehreren inaktivierten Virusflüssigkeiten vermischt wird, die abgeleitet sind von einem gänzlich verschiedenen Virustyp, wie

**0 030 063**

Newcastle Disease-Virus, einem Adenoähnlichen Virus und/oder Reo-Virus.

10. Infektiöse-Bronchitis-Lebend-Vakzinen, erhalten nach den Verfahren der Ansprüche 1 bis 8.

11. Inaktivierte infektiöse-Bronchitis-Vakzinen, erhalten gemäß den Verfahren der Ansprüche 1 bis 9.

12. Verfahren zur Herstellung eines IBV—Stammes eines neuen Serotyps, gekennzeichnet durch die interne Bezeichnung Utrecht.101, Utrecht.102, Drente.201, Limburg.501, Limburg.502, Brabant.801, Limburg.536, Overijssel.728 und Utrecht.121, welche Stämme in der Tschechoslowakischen Nationalen Kultursammlung, Institut für Hygiene und Epidemiologie in Prag unter den Nummern CNCTC A07/80, CNCTC A08/80, CNCTC A09/80, CNCTC A010/80, CNCTC A011/80, CNCTC A016/80, CNCTC A014/80, CNCTC A015/80 und CNCTC A013/80 und in der Collection Nationale de Cultures de Microorganismes d'Institut Pasteur, Paris, unter den Nummern I.111, I.112, I.113, I.109, I.110, I.132, I.134, I.135 und I.133 hinterlegt worden sind, durch Isolieren mit Hilfe der Luftröhrenabstrichmethode bei Scharen von Hühnern, die mit der üblichen IBV H120 und/oder H52-Vakzine geimpft worden sind und im Vergleich zum virus des Massachussets-Typs hohe humorale Antikörpertiter beim Beginn des Auftretens von Respirationssymptomen und/oder einem Abfall der Eiproduktion aufweisen, oder bei Brathühnern, die in der zweiten Hälfte der Mastperiode nach Impfung mit einer IBV—Vakzine des H-Typs Atmungssymptome zeigten, und Attenuation eines solchen Virus an SPF-Hühnerembryonen.

**Revendications pour les etats contractants: BE CH LI DE FR GB IT LU NL SE**

1. Vaccinscontre la bronchite infectieuse pour la volaille, caractérisés ence qu'ils dérivent d'au moins une souche de virus d'un nouveau sérotype des virus de la bronchite infectieuse identifiées par les notations internes Utrecht.101, Utrecht.102, Drente.201, Limburg.501, Limburg.502, Brabant.801, Limburg.536, Overijssel.728 et Utrecht.121, qui ont été déposées au Czechoslovak National Collection of Type Cultures de i'Institut d'Hygiène et d'Epidémiologie à Prague sous les numéros CNCTC A 07/80, CNCTC A 08/80, CNCTC A 09/80, CNCTC A 010/80, CNCTC A 011/80, CNCTC A 016/80, CNCTC A 014/80, CNCTC A 015/80 et CNCTC A 013/80; et à la Collection Nationale de Cultures de Microorganismes de l'Institut Pasteur à Paris sous les numéros respectifs I—111, I—112, I—113, I—109, I—110, I—132, I—134, I—135 et I—133.

2. Vaccins contre la bronchite infectieuse suivant la revendication 1, caractérisés en ce qu'ils dérivent d'au moins une souche de virus d'un nouveau sérotype du virus de la bronchite infectieuse identifiées par les notations internes Utrecht.101, Limburg.536 et Overijssel.728 qui ont été déposées au Czechoslovak National Collection of Type Cultures de l'Institut d'Hygiène et d'Epidemiologie à Prague sous les numéros CNCTC A 07/80, CNCTC A 014/80 et CNCTC A 015/80 et à la Collection Nationale de Cultures de Microorganismes de l'Institut Pasteur sous les numéros I—111, I—134 et I—135.

3. Vaccinscombinés contre la bronchite infectieuse suivant les revendications 1 et 2, caractérisé en ce que ces vaccins dérivent en outre de l'IBV H120 ou de l'IBV H52 du type Massachussetts.

4. Vaccin contre la bronchite infectieuse suivant la revendication 3, caractérisé en ce qu'il dérive en outre de l'IBV H52 du type Massachussetts.

5. Vaccins vivants contre la bronchite infectieuse suivant la revendication 4, caractérisé en ce qu'ils manifestent après lyophilisation une teneur en virus de $\geqslant 10^{4,0}$ $EID_{50}$ par dose.

6. Vaccins vivants contre la bronchite infectieuse suivant la revendication 5, caractérisé en ce qu'ils manifestent après lyophilisation une teneur totale en virus de $\geqslant 2 \times 10^{4,0}$ $EID_{50}$ par dose de chaque souche de virus.

7. Vaccin inactivé contre la bronchite infectieuse suivant les revendications 1—4, caractérisé en ce qu'il contient une phase huileuse contenant comme constituants essentiels au moins une huile minérale ou végétale et un ou plusieurs agents émulsionnants appropriés sous la forme d'un agent tensio-actif non ionogène issu d'un oxyde d'alcoylène et/ou d'alcools hexahydroxylés et/ou d'acides gras naturels supérieurs de 10 à 20 atomes de carbone, comme des esters ou ester-éthers.

8. Souches de virus de la bronchite infectieuse d'un nouveau sérotype identifiées par les notations internes Utrecht.101, Utrecht.102, Drente.201, Limburg.501, Limburg.502, Brabant.801, Limburg.536, Overijssel.728 et Utrecht.121 qui ont été déposées au Czechoslovak National Collection of Type Cultures de l'Institut d'Hygiène et d'Epidémiologie à Prague sous la numéros CNCTC A 07/80, CNCTC A 08/80, CNCTC A 09/80, CNCTC A 010/80, CNCTC A 011/80, CNCTC A 016/80, CNCTC A 014/80, CNCTC A 015/80 et CNCTC A 013/80 et à la Collection Nationale de Cultures de Microorganismes de l'Institut Pasteur à Paris sous les numéros respectifs I—111, I—112, I—113, I—109, I—110, I—132, I—134, I—135 et I—133.

9. Souches de virus de la bronchite infectieuse identifiées par les notations Utrecht.101, Limburg.536 et Overijssel.728 et déposées au Czechoslovak National Collection of Type Cultures de l'Institut d'Hygiene et d'Epidémiologie à Prague sous les numéros CNCTC A 07/80, CNCTC A 014/80 et CNCTC A 015/80 et à la Collection Nationale de Cultures de Microorganismes de l'Institut Pasteur à Paris sous les nos I—111, I—134 et I—135.

10. Procédé de préparation de vaccins contre la bronchite infectieuse pour la volaille suivant la

13

revendication 1, caractérisé en ce qu'on propage au moins une des souches de virus comme virus d'ensemencement dans des oeufs de poule fécondés ou dans des cultures de cellules appropriées, comme des cultures de cellules de rein de poulet SPF, après quoi on récolte le matériel viral cultivé et on le purifie, puis on lyophilise le mélange après addition éventuelle de stabilisants et/ou d'antibiotiques.

11. Procédé suivant la revendication 10, caractérisé en ce qu'on inocule le virus d'ensemencement, dans des conditions stérles, dans la cavité allantoïque d'oeufs de poule SPF type I qui ont été préincubés pendant 10 à 11 jours, après quoi on récolte le liquide amnio-allantoïque des embryons encore vivants et spécifiquement morts après incubation à +37°C penant 28 à 34 heures, on le purifie et après addition de stabilisants et éventuellement d'antibiotiques, on le lyophilise.

12. Procédé suivant les revendications 10 et 11, caractérisé en ce qu'on prépare des vaccins simples qui contiennent, après lyophilisation, $\geqslant 10^{4,0}$ EID$_{50}$ par dose du virus concerné.

13. Procédé de préparation de vaccins combinés suivant les revendications 3 et 4, caractérisé en ce qu'on mélange le liquide viral, obtenu par culture d'au moins une souche de virus des nouveaux sérotypes des virus de la bronchite infectieuse identifiés par les notations internes Utrecht.101, Limburg.501, Limburg.502, Utrecht.102, Drente.201, Brabant.801, Limburg.536, Overijssel.728 et Utrecht.121 et ensuite purification et mélange, après addition éventuelle de stabilisants et/ou d'antibiotiques, avec le liquide viral obtenu par culture d'un IBV H120 ou d'un IBV H52, purification et addition éventuelle de stabilisants et d'antibiotiques éventuels, puis on les lyophilise.

14. Procédé de préparation de vaccins combinés suivant la revendication 13, caractérisé en ce qu'on prépare des vaccins qui ont, après lyophilisation, une teneur en virus $\geqslant 10^{4,0}$ EID$_{50}$ par dose et de préférence $\geqslant 2 \times 10^{4,0}$ EID$_{50}$ par dose de chaque constituant viral.

15. Vaccin inactivé contre la bronchite infectieuse suivant la revendication 7, caractérisé en ce qu'il dérive, outre d'une ou plusieurs souches IBV, aussi d'un ou plusieurs types de virus totalement différents comme le virus de la maladie de Newcastle, un adéno virus et/ou un réo virus.

**Revendications pour l'Etat Contractant: AT**

1. Procédé de préparation de vaccins contre la bronchite infectieuse pour la volaille, caractérisé en ce qu'on propage au moins une souche de virus d'un nouveau sérotype des virus de la bronchite infectieuse identifiés par les notations internes Utrecht.101, Utrecht.102, Drente.201, Limburg.501, Limburg.502, Brabant.801, Limburg.536, Overijssel.728 et Utrecht.121, qui ont été déposés au Czechoslovak National Collection of Type Cultures de L'institut d'Hygiène et d'Epidémiologie à Prague sous les numéros CNCTC A 07/80, CNCTC A 08/80, CNCTC A 09/80, CNCTC A 010/80, CNCTC A 011/80, CNCTC A 016/80, CNCTC A 014/80, CNCTC A 015/80 et CNCTC A 013/80, et à la Collection Nationale de Cultures de Microorganismes de l'Institut Pasteur à Paris sous les numéros respectifs I—111, I—112, I—113, I—109, I—110, I—132, I—134, I—135 et I—133 comme virus d'ensemencement suivant des techniques connues, après quoi on récolte le matériel viral cultivé et on le purifie, puis on lyophilise le mélange éventuellement après addition de stabilisants et/ou d'antibiotiques et/ou inactivation de manière connue.

2. Procédé suivant la revendication 1, caractérisé en ce qu'on propage le virus d'ensemencement dans des oeufs de poule fécondés ou dans des cultures de cellules appropriées, par exemple des cultures de cellules de rein de poulet SPF.

3. Procédé suivant les revendications 1 et 2, caractérisé en ce qu'on inocule le virus d'ensemencement, dans des conditions stériles, dans la cavité allantoïque d'oeufs de poule SPF type I qui ont été pré-incubés pendant 10 à 11 jours, après quoi on récolte le liquide amnio-allantoïque des embryons encore vivants et spécifiquement morts après incubation à +37°C pendant 28 à 34 heures, on le purifie et après addition de stabilisants et éventuellement d'antibiotiques, on le lyophilise.

4. Procédé suivant les revendications 1—3, caractérisé en ce qu'on prépare des vaccins simples qui contiennent, après lyophilisation, $\geqslant 10^{4,0}$ EID$_{50}$ par dose du virus concerné.

5. Procédé suivant les revendications 1—4, caractérisé en ce qu'on prépare des vaccins issus des souches IBV Utrecht.101, Limburg.536 ou Overijssel.728.

6. Procédé de préparation de vaccins combinés suivant les revendications 1—5, caractérisé en ce qu'on mélange le liquide viral, obtenu par propagation d'au moins une souche de virus des nouveaux sérotypes des virus de la bronchite infectieuse identifiés par les notations internes Utrecht.101, Limburg.501, Limburg.502, Utrecht.102, Drente.201, Brabant.801, Limburg.536, Overijssel.728 et Utrecht.121 et ensuite purification, après addition eventuelle de stabilisants et/ou d'antibiotiques, avec un liquide viral obtenu par culture d'un IBV H120 ou d'un IBV H52, purification et addition éventuelle de stabilisants et d'antibiotiques éventuels, puis inactivation et/ou lyophilisation.

7. Procédé de préparation de vaccins combinés suivant la revendication 6, caractérisé en ce qu'on prépare des vaccins qui ont, après lyophilisation, une teneur en virus $\geqslant 10^{4,0}$ EID$_{50}$ par dose de chaque constituant viral.

8. Procédé de préparation de vaccins combinés suivant les revendications 6—7, caractérisé en ce que les vaccins dérivent d'au moins une souche IBV choisie entre Utrecht.101, Limburg.536 et Overijssel.728.

14

9. Procédé de préparation de vaccins inactivés combinés prêts à l'utilisation, caractérisé en ce qu'on mélange, après inactivation, un liquide viral issu d'une ou plusieurs des souches de virus IBV des nouveaux sérotypes avec un ou plusieurs liquides viraux inactivés issus d'un type de virus totalement différent comme le virus de la maladie de Newcastle, un adéno virus et/ou un réo virus.

10. Vaccins vivants contre la bronchite infectieuse, obtenus par les procédés suivant les revendications 1—8.

11. Vaccins inactivés contre la bronchite infectieuse, obtenus par les procédés suivant les revendications 1—9.

12. Procédé de préparation d'une souche IBV d'un nouveau sérotype identifiée par les notations internes Utrecht.101, Utrecht.102, Drente.201, Limburg.501, Limburg.502, Brabant.801, Limburg.536, Overijssel.728 et Utrecht.121 qui ont été déposées au Czechoslovak National Collection of Type Cultures de l'Institut d'Hygiène et d'Epidémiologie à Prague sous les numéros CNCTC A 07/80, CNCTC A 08/80, CNCTC A 09/80, CNCTC 010/80, CNCTC A 011/80, CNCTC A 016/80, CNCTC A 014/80, CNCTC A 015/80 et CNCTC A 013/80 et à la Collection Nationale de Cultures de Microorganismes de l'Institut Pasteur à Paris sous les numéros respectifs I—111, I—112, I—113, I—109, I—110, I—132, I—134, I—135 et I—133 par isolement suivant la technique de frottis de trachée sur des groupes de poules qui ont été vaccinées au moyen du vaccin IBV H.120 et/ou H.52 habituel et qui présentent des titres d'anticorps humoraux élevés du virus type Massachussetts au début de l'apparition des symptômes respiratoires et/ou de la baisse de ponte, ou sur des poulets de chair à la seconde moitié de la période d'engraissement qui manifestent des symptômes respiratoires après vaccination au moyen d'un vaccin IBV de type H, et par atténuation de ces virus sur des embryons de poulet SPF.